# EUROPEAN PATENT APPLICATION

(11) **EP 4 056 555 A1**
(43) Date of publication of application: **14.09.2022**
(21) Application number: 21798929.2
(22) Date of filing: 18.05.2021
(51) Int. Cl.: C07C 405/00, A61K 31/216, A61P 27/06

(54) **METHOD FOR PURIFYING TAFLUPROST**

(30) Priority: 27.01.2021 CN 202110111199
(71) Applicant: AGC Inc., Tokyo 100-8405 (JP)
(72) Inventor: KONISHI, Katsuhiko, Tokyo 100-8405 (JP); MORI, Nobuaki, Tokyo 100-8405 (JP); MATSUMURA, Yasushi, Tokyo 100-8405 (JP)
(74) Representative: Müller-Boré & Partner Patentanwälte PartG mbB
(86) International application number: PCT/JP2021/018791
(87) International publication number: WO 2022/162967

(57) **Abstract**

The present invention aims to provide a purification method of tafluprost which is convenient, efficient, and can withstand scale-up. The present invention relates to a method for purifying tafluprost, including a step of purifying a crude product of tafluprost by silica gel column chromatography, and collecting a fraction containing tafluprost by HPLC analysis. The present invention also relates to a production method of tafluprost, including the aforementioned purification method of tafluprost.

## Description

### [Technical Field]

The present invention relates to a novel purification method of tafluprost.

### [Background Art]

Tafluprost is represented by the following formula:

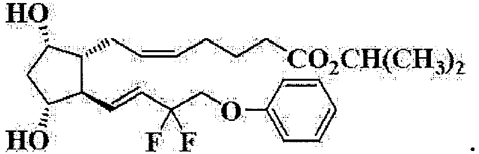

It has a chemical name of isopropyl (5Z)-7-[(1R,2R,3R,5S)-2-[(1E)-3,3-difluoro-4-phenoxy-1-butenyl]-3,5-dihydroxycyclopentyl]-5-heptenoate, and is an extremely highly viscous difluoroprostaglandin F_{2α} derivative having a viscosity of 2440 mPa·s at 25°C. Tafluprost has an unstable chemical structure having two double bonds, an unsaturated fatty acid ester moiety, and four asymmetric centers, in which a hydroxy group at the C15 position and a hydrogen atom present in other prostaglandin derivatives are substituted by two fluorine atoms, due to which it shows specific physical property of remarkably high lipophilicity among the prostaglandin derivatives, and also shows property of high chemical stability as a prostaglandin derivative, though decomposition occurs at a high temperature. In addition, tafluprost has a potent intraocular pressure-lowering action, and is used as an eye drop for the treatment of glaucoma and ocular hypertension (patent document 1). Patent document 1 describes a method for producing a difluoroprostaglandin F_{2α} derivative including tafluprost, and non-patent document 1 also describes a similar production method.

In the production method described in patent document 1, contamination by an α chain trans isomer in the final product is difficult to avoid because the method includes a Wittig reaction step. In the production method described in patent document 1, a method for separation and purification by preparative HPLC (high performance liquid chromatography) has been reported as a method for removing impurities including α chain trans isomers (patent document 2). However, tafluprost, and a carboxylic acid compound represented by the following formula (I):

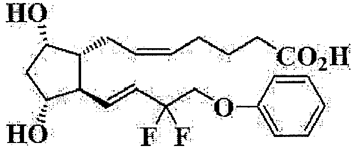

(hereinafter to be referred to as "tafluprost acid"), which is a synthesis precursor for tafluprost, are both oily compounds having an extremely high viscosity, and are difficult to purify. Moreover, since a large amount of organic solvent is used in the purification method of tafluprost described in patent document 2, a large cost is required, and it is difficult to suppress the concentration of the residual organic solvent below the concentration limit value indicated in the Residual Solvent Guideline for Pharmaceutical Product (non-patent document 2). The columns for preparative HPLC are generally expensive and are usually used repeatedly, which causes problems such as contamination by accumulated impurities and decomposition products and a decrease in the number of theoretical stages due to the deterioration of the column. To reduce the risk caused by these problems, complicated validation with regard to washing with a large amount of organic solvent and confirmation thereof, confirmation of column separation ability, and the like is also routinely required. Thus, this method is not practical as a method for producing pharmaceutical products.

On the other hand, methods for reducing contamination by impurities such as an α-chain trans isomer and the like via an organic amine salt (patent document 3, 4) or a metal salt (patent document 5) of tafluprost acid have also been reported. However, along with the addition of a salt formation step and a step of dissociation from the salt, by-products, dehydrating products, other impurities, and the like due to dimerization by condensation with organic amine or self-condensation may increase. In addition, many organic amines and metals are feared for toxicity, mutagenicity, and the like, and it is problematic in view of safety to use them in a purification method particularly at a stage close to the final step of pharmaceutical products.

Furthermore, a production method of tafluprost that prevents contamination by the α-chain trans isomer by undergoing the steps of forming a macrolactone ring and opening the macrolactone ring has also been reported (patent document 6). However, such production method is not practical because the production process is long and the yield is low.

### [Document List]

### [Patent documents]

Patent document 1: EP 0850926 A2
Patent document 2: US 2014/0051882 A1
Patent document 3: WO 2013/118058 A1
Patent document 4: WO 2016/090461 A1
Patent document 5: CN 108299192 A
Patent document 6: JP-A-2015-36382

### [Non-patent documents]

Non-patent document 1: Tetrahedron Lett., 2004, 45, 1527-1529
Non-patent document 2: Notification No. 307 (by the Director) of Evaluation and Licensing Division, Pharmaceutical and Medical Safety Bureau, Ministry of Health and Welfare (March 30, 1998), Guideline Residual Solvent in Pharmaceutical Product

### [Summary of Invention]

### [Technical Problem]

The present invention aims to provide a purification method of tafluprost that can purify tafluprost, which is an oily compound with extremely high viscosity, conveniently at a low cost to a purity of a level permitting use as it is as a drug substance of pharmaceutical products, and that can be scaled up.

### [Solution to Problem]

The present inventors have conducted intensive studies in an attempt to solve the above-mentioned problems and found that tafluprost with a high purity can be obtained by a purification method of tafluprost including a step of purifying a crude product of tafluprost, which is obtained by an esterification step of tafluprost acid, by silica gel column chromatography, and collecting a fraction containing tafluprost by HPLC analysis (hereinafter sometimes to be referred to as "the purification method of the present invention"). They have also found that the concentration of the residual organic solvent can be suppressed below the concentration limit value indicated in the Residual Solvent Guideline for Pharmaceutical Products by performing a purification method including a step of concentrating tafluprost-containing fractions collected by HPLC analysis under reduced pressure at 10 - 55°C, a step of dissolving the residue in a solvent and filtering the solution, and a step of evaporating the solvent from the filtrate at 10 - 55°C under reduced pressure that affords a final ultimate vacuum of 5 torr or less (hereinafter a purification method including all the aforementioned steps is sometimes to be referred to as "the purification method of the present invention"), and that tafluprost having a purity permitting provision thereof as a drug substance of pharmaceutical products can be obtained, which resulted in the completion of the present invention.

Accordingly, the present invention provides the following.
[1] A method for purifying tafluprost, comprising a step of purifying a crude product of tafluprost by silica gel column chromatography, and collecting a fraction containing tafluprost by HPLC analysis.
[2] The purification method of the above-mentioned [1], further comprising a step of concentrating the tafluprost-containing fraction collected by the HPLC analysis under reduced pressure at 10 - 55°C, a step of dissolving the residue in a solvent and filtering the solution, and a step of evaporating the solvent from the filtrate at 10 - 55°C under reduced pressure that affords a final ultimate vacuum of 5 torr or less.
[3] The purification method of the above-mentioned [1] or [2], wherein a particle size (d50) of silica gel used for the silica gel column chromatography is 20 - 70 µm.
[4] The purification method of any of the above-mentioned [1] to [3], wherein the silica gel used for the silica gel column chromatography is spherical.
[5] The purification method of any of the above-mentioned [1] to [4], wherein an eluent of the silica gel column chromatography is a mixed solvent of n-hexane and a polar solvent, or a mixed solvent of n-heptane and a polar solvent.
[6] The purification method of the above-mentioned [5], wherein the eluent is a mixed solvent of n-hexane and a polar solvent.
[7] The purification method of the above-mentioned [5] or [6], wherein the polar solvent is ethyl acetate, t-butyl methyl ether, 2-propanol, or ethanol.
[8] The purification method of any of the above-mentioned [1] to [7], wherein the HPLC analysis is reversed phase HPLC analysis.
[9] The purification method of any of the above-mentioned [1] to [8], wherein the fraction comprises tafluprost in a proportion of 98% or more.
[10] The purification method of any of the above-mentioned [2] to [9], wherein the filtration is performed using a filter having a pore size of 0.5 µm or less.
[11] The purification method of any of the above-mentioned [2] to [10], wherein the solvent used for dissolving the residue is ethyl acetate, t-butyl methyl ether, 2-propanol or ethanol, or a mixed solvent of a nonpolar solvent and ethyl acetate, t-butyl methyl ether, 2-propanol or ethanol.
[12] The purification method of the above-mentioned [11], wherein the solvent used for dissolving the residue is ethyl acetate or a mixed solvent of ethyl acetate and a nonpolar solvent.
[13] The purification method of the above-mentioned [11] or[12],
   wherein the nonpolar solvent is n-hexane or n-heptane.
[14] The purification method of any of the above-mentioned [2] to [13], wherein the final ultimate vacuum is 1 torr or less.
[15] The purification method of any of the above-mentioned [2] to [14], wherein, after the step of evaporating the solvent from the filtrate, the residual solvent concentration of n-hexane is 290 ppm or less, and the residual solvent concentration of each of n-heptane, ethyl acetate, t-butyl methyl ether, 2-propanol and ethanol is 5000 ppm or less.
[16] A method for producing tafluprost, comprising a step of subjecting a crude product of tafluprost to the purification method of any of the above-mentioned [1] to [15].
[17] A tafluprost obtained by the production method of the above-mentioned [16].
[18] A medicament comprising the tafluprost of the above-mentioned [17] as an active ingredient.
[19] A medicament for the prophylaxis or treatment of an ophthalmic disease, comprising the tafluprost of the above-mentioned [17] as an active ingredient.
[20] The medicament of the above-mentioned [19], wherein the ophthalmic disease is glaucoma or an ocular hypertension.

### [Effect of Invention]

According to the purification method of the present invention, the contamination by impurities can be minimized by collecting fractions containing tafluprost by HPLC analysis during separation and purification of a crude product of tafluprost by silica gel column chromatography in the final step of tafluprost production. In addition, the concentration of the residual organic solvent can be suppressed below the concentration limit value indicated in the Residual Solvent Guideline for Pharmaceutical Products by evaporating the solvent over time under reduced pressure conditions of low temperature and high vacuum level, the decomposition of tafluprost which is unstable under high temperature can also be suppressed. Furthermore, fine powder of silica gel, airborne particles in the air, and bacteria can be removed by incorporating a filter filtration step thereinto. After evaporation of the solvent, therefore, it is possible to easily and efficiently provide highly pure tafluprost that can be used as it is as a drug substance of pharmaceutical products. The purification method of the present invention can be widely applied to the crude products of tafluprost produced by known methods, and can withstand scale-up.

### [Description of Embodiments]

The embodiments of the present invention are explained in detail in the following.

### [Definition of terms]

The terms in the present specification mean the following.

In the present specification, both an open column and a flash column can be used as the column to be used for "silica gel column chromatography".

The "silica gel column chromatography" in the present specification is a normal phase column chromatography.

In the present specification, the "crude product of tafluprost" means a product after work-up of the reaction and before purification in the final step in a known production method of tafluprost. Specifically, it includes, for example, the resultant product before purification in the final esterification reaction in the production method of tafluprost described in patent document 1, as shown in the Examples described later.

In the present specification, the "impurity" encompasses all substances other than tafluprost, such as residual reagents, residual starting material compounds, by-products of the reaction, analogous substances such as decomposition products of tafluprost, and the like, as well as residual organic solvents, filler-derived residues, bacteria, and the like, contained in the aforementioned crude product of tafluprost.

In the present specification, the "HPLC analysis" means confirmation of the presence or absence and the content ratio of tafluprost in each fraction by using high performance liquid chromatography for analysis when separating and purifying a crude product of tafluprost by silica gel column chromatography.

In the present specification, the "filtration" means filter filtration. The filtration is performed for the purpose of removing fine powder of column filler (silica gel), airborne particles, bacteria and the like.

In the present specification, the "polar solvent" means a solvent with a high dielectric constant. Specific examples of the polar solvent include esters such as ethyl acetate, propyl acetate, and the like, ethers such as diethyl ether, t-butyl methyl ether, tetrahydrofuran, and the like, alcohols such as 2-propanol, ethanol, and the like, and the like. Among these, ethyl acetate, t-butyl methyl ether, 2-propanol or ethanol is preferred.

In the present specification, the "nonpolar solvent" means a solvent with a low dielectric constant. Specific examples of the nonpolar solvent include chain hydrocarbons such as n-hexane, n-heptane and the like. Of these, n-hexane is preferred.

In the present specification, the "outer temperature" means the temperature outside the reaction vessel or concentration container, which is generally the outside temperature or the temperature of a water bath or hot water bath.

In the present specification, the "concentration limit value indicated in the Residual Solvent Guideline for Pharmaceutical Products" is a value of a permissible amount of the residual solvent in pharmaceutical products which was considered as one of the issues of the Japan-U.S.-EU Trilateral International Council for Harmonisation of Technical Requirements for Pharmaceuticals for Human Use (ICH), and specified for the safety of patients, and means a toxicologically acceptable limit of solvent residues. Specific examples of the concentration limit value of residual solvents in pharmaceutical products include, as described in non-patent document 2, 290 ppm for n-hexane, and 5000 ppm for n-heptane, ethyl acetate, t-butyl methyl ether, 2-propanol or ethanol.

### [Purification method of the present invention]

The purification method of the present invention characteristically includes a step of purifying a crude product of tafluprost by silica gel column chromatography, and collecting a fraction containing tafluprost by HPLC analysis (step 1). To suppress the concentration of the residual organic solvent below the concentration limit value indicated in the Residual Solvent Guideline for Pharmaceutical Products, moreover, the purification method of the present invention characteristically includes, in addition to the aforementioned step 1, a step of concentrating under reduced pressure at 10 - 55°C (step 2), a step of dissolving the residue in a solvent and filtering the solution (step 3), and a step of evaporating the solvent from the filtrate at 10 - 55°C under reduced pressure that affords a final ultimate vacuum of 5 torr or less (step 4).

### (Step 1)

In this step, purification is performed by silica gel column chromatography, and fractions containing tafluprost are collected by HPLC analysis.

The filler to be used for silica gel column chromatography is not particularly limited as long as it can be used for a general normal phase column. The shape of the silica gel may be crushed or spherical, and more preferably spherical. The particle size (d50) of the silica gel is not particularly limited. It is preferably 20 µm - 70 µm, more preferably 40 µm - 65 µm, particularly preferably 45 µm - 60 µm. The particle size (d50) is the median diameter of the particle size distribution when the particle size distribution is prepared on a volume basis by laser diffraction scattering type particle size distribution measurement.

The eluent to be used for silica gel column chromatography is not particularly limited as long as it is a solvent capable of separating tafluprost and impurities in a crude product of tafluprost, and is a mixed solvent of n-hexane and a polar solvent, preferably a mixed solvent of heptane and a polar solvent, and more preferably a mixed solvent of n-hexane and a polar solvent. The polar solvent is selected from ethyl acetate, t-butyl methyl ether, 2-propanol and ethanol, and 2-propanol or ethanol is preferable. The mixing ratio (volume ratio) of n-hexane and a polar solvent, or n-heptane and a polar solvent may be appropriately determined according to the kind, shape, and/or particle size of the filler to be used. Specific preferable examples of the eluent include ethyl acetate, t-butyl methyl ether, a mixed solvent of 2-propanol or ethanol, and a nonpolar solvent (preferably, n-hexane or n-heptane), more preferably, a mixed solvent of 2-propanol or ethanol, and a nonpolar solvent (preferably, n-hexane or n-heptane), particularly preferably a mixed solvent of ethanol and n-hexane. When a mixed solvent is used as an eluent, the mixing ratio (volume ratio) is not particularly limited. From the aspect of controlling the residual solvent concentration to below the standard value, in the case of a mixed solvent of ethanol and n-hexane, it is preferable to use a solvent in which ethanol:n-hexane are mixed at 10:90 to 1:99, more preferably a solvent in which ethanol:n-hexane are mixed at 6:94 to 2:98, further preferably a solvent in which ethanol:n-hexane are mixed at 5:95 - 3:97, particularly preferably a solvent in which ethanol:n-hexane are mixed at 4:96.

HPLC for analysis is used to confirm the presence or absence of tafluprost in the fractions separated by silica gel column chromatography purification. As the HPLC, both normal phase HPLC and reversed phase HPLC can be used, but reversed phase HPLC, which is superior in terms of impurity separation efficiency, detection sensitivity, quantitativity, and the like, is more preferable. Specific examples of the column and analysis conditions to be used in the HPLC analysis include, but are not limited to, the conditions in the Examples described later.

Generally, when performing silica gel column chromatography purification, the presence or absence of the desired product in the separated fraction is confirmed using TLC (thin layer chromatography) *(*Jikken Kagaku Kouza 1 Basic Operation I (4th Edition), published on November 5, 1990, MARUZEN, 5.2.3 Column Chromatography, p. 293-296). In purifying a crude product of tafluprost, HPLC analysis was found to be remarkably superior in detection sensitivity of fractions containing tafluprost and impurities as compared with conventional TLC analysis.

Silica gel column chromatography was performed on many lots of the crude product of the synthesized tafluprost, and the elution pattern of impurities was analyzed by HPLC analysis. As a result, it was confirmed that the elution pattern of the impurities was always constant. By consideration of such elution pattern of the impurities, it was found to be preferable to collect continuous fractions in which the HPLC area percentage of tafluprost in each fraction is 97% or more, particularly preferably 98% or more.

### (Step 2)

In this step, fractions containing tafluprost as confirmed by HPLC analysis are collected and concentrated under reduced pressure at 10 - 50°C.

The outer temperature (temperature of water bath or hot-water bath) during concentrating the collected fractions containing tafluprost as confirmed by HPLC analysis under reduced pressure is preferably 10°C - 55°C, more preferably 15°C - 50°C, particularly preferably 20 - 45°C. As shown in the Experimental Examples described later, it was confirmed that tafluprost gradually decomposes over time at a temperature of 60°C or more. Thus, it is desirable to concentrate under reduced pressure or evaporate the solvent at the above-mentioned temperature.

### (Step 3)

In this step, the residue obtained in the aforementioned step 2 is dissolved in a solvent and filtered. Since tafluprost has extremely high viscosity, it is difficult to perform sterilization filtration after completely evaporating the solvent. Therefore, the purification method of the present invention is characterized by incorporation of a filtration step after step 2.

Examples of the solvent for dissolving the residue obtained in step 2 include solvents similar to the eluents used for silica gel column chromatography in step 1. A solvent that sufficiently dissolves tafluprost and has a relatively low boiling point is preferred, and a mixed solvent with a nonpolar solvent forming an azeotropic composition may also be used. Specific preferable examples include ethyl acetate, t-butyl methyl ether, 2-propanol or ethanol, or a mixed solvent of ethyl acetate, t-butyl methyl ether, 2-propanol or ethanol, and a nonpolar solvent (preferably, n-hexane or n-heptane), more preferably, a mixed solvent of ethyl acetate or ethyl acetate, and a nonpolar solvent (preferably, n-hexane or n-heptane), particularly preferably, a mixed solvent of ethyl acetate and n-hexane. When a mixed solvent is used, the mixing ratio (volume ratio) is not particularly limited. From the aspect of controlling the residual solvent concentration to below the standard value, in the case of a mixed solvent of ethyl acetate and n-hexane, it is particularly preferable to use a solvent in which ethyl acetate:n-hexane are mixed at 10:1 - 1:10, preferably 4:1 - 1:4, more preferably 2:1 - 1:2.

The filter to be used for the filtration in this step is not particularly limited as long as it does not swell or dissolve in a solvent and can remove fine powder of a filler (silica gel), air borne particles in the air, and the like. For example, glass fiber filter, polypropylene filter, nylon filter, fluorine resin filter and the like can be mentioned, and filter of fluorine resin such as polyvinylidene difluoride (PVDF), polytetrafluoroethylene (PTFE) and the like is preferred. Among these, polytetrafluoroethylene (PTFE) filter is particularly preferred.

The pore size of the filter is generally 0.5 µm or less, and preferably 0.25 µm or less. When sterilization is also desired, the pore size is particularly preferably 0.22 µm or less.

### (Step 4)

In this step, the solvent of the filtrate obtained the aforementioned step 3 is evaporated at 10 - 55°C under reduced pressure that affords a final ultimate vacuum of 5 torr or less.

Since tafluprost has extremely high viscosity, it is necessary to make the surface area from which the solvent evaporates as large as possible and gradually evaporate the solvent of the filtrate over time while avoiding bumping. Examples of an apparatus for concentration under reduced pressure for achieving such purpose include a rotary evaporator, a centrifugal evaporator, a high vacuum thin film distillation apparatus, and the like.

As described above, tafluprost gradually decomposes over time at a temperature of 60°C or more. Thus, the outer temperature (temperature of water bath or hot-water bath) during evaporation of the solvent under reduced pressure is preferably 10°C - 55°C, more preferably 15°C - 50°C, particularly preferably 20°C - 45°C.

The degree of reduced pressure when evaporating the solvent is preferably controlled such that the final ultimate vacuum is 5 torr or less (preferably, 3 torr or less, more preferably 1 torr or less, particularly preferably 0.5 torr or less) by making the surface area from which the solvent evaporates as large as possible and gradually increasing the degree over time while avoiding bumping. When breaking the reduced pressure, it is preferable to return to normal pressure by using filtered air in order to prevent the invasion of airborne particles and bacteria in the air.

The time for evaporating the solvent is preferably 10 - 70 hr, more preferably 15 - 60 hr, particularly preferably 20 - 60 hr.

Using the purification method of the present invention, the concentration of the residual organic solvent can be suppressed below the concentration limit value indicated in the Residual Solvent Guideline for Pharmaceutical Products (non-patent document 2). While the guideline states, "all residual solvents should be reduced below levels that can meet product standards, GMP or other quality standards, since residual solvents are not useful for treatment", it is possible to stably produce tafluprost with high quality that can meet various more stringent quality standards.

The residual solvent concentration of ethyl acetate, t-butyl methyl ether, 2-propanol, ethanol, or n-heptane exemplified as preferable solvents as eluent to be used for the aforementioned silica gel column chromatography and solvent to dissolve residue is more preferably controlled to 1000 ppm or less, particularly preferably 100 ppm or less, during production. In addition, the residual solvent concentration of n-hexane is more preferably controlled to 200 ppm or less, particularly preferably 20 ppm or less, during production. The residual solvent concentration can be measured by a method such as gas chromatography (GC) and the like.

The present invention also encompasses a production method of tafluprost, including a step of subjecting a crude product of tafluprost produced by a known method to the purification method of the present invention (purification method including the above-mentioned steps 1 - 4). Known production methods of tafluprost include the aforementioned patent document 1 and non-patent document 1, and some reports (e.g., US 2014/0046086 A1; J. Org. Chem. 2016, 81, 10832-844; Molecules, 2017, 22, 217, 1-16; Org. Lett. 2020, 22, 2991-2994, etc.), and these are also combined with the purification method of the present invention and encompassed in the present invention.

Specific examples of the crude product of tafluprost to be used in the present invention include a crude product of tafluprost which is obtained by a deprotection reaction from tafluprost in which a hydroxy group and the like are protected, a crude product of tafluprost obtained by esterification of a salt of tafluprost acid, a crude product of tafluprost obtained by esterification of tafluprost acid, and the like. Among these, a crude product of tafluprost obtained by esterification of tafluprost acid is preferably used.

Specific characteristics of the purification method of the present invention include the following.
(A) The contamination by impurities can be minimized by collecting fractions containing tafluprost by HPLC analysis (preferably, reversed phase HPLC analysis) during separation and purification of a crude product of tafluprost by silica gel column chromatography.
(B) The decomposition of tafluprost which is unstable under high temperature can be suppressed, and the concentration of the residual organic solvent can be suppressed below the concentration limit value indicated in the Residual Solvent Guideline for Pharmaceutical Products, by evaporating the solvent over time under reduced pressure conditions of low temperature and high vacuum level.
(C) After evaporation of the solvent, highly pure tafluprost that can be used as it is as a drug substance of pharmaceutical products can be provided by incorporating a filter filtration step thereinto.
(D) The purification method of the present invention can also be applied to a crude product of tafluprost obtained by using any of the known production methods of tafluprost, and can be easily scaled up. Therefore, a convenient and highly efficient purification method can be provided.

As described in the above-mentioned (A), when the purification method of the present invention is performed for the purpose of increasing the purity of tafluprost, it is sufficient to include only the above-mentioned step 1, and each of steps 2 - 4 can also be performed in combination with step 1 as necessary.

### [Example]

The present invention is explained in detail in the following by referring to Reference Example, Examples and Experimental Example, which are not to be construed as limitative.

Unless otherwise specified, % shows mol% for yield and mass% for others. The ratio shown in the mixed solvent indicates the volume ratio unless otherwise specified. The room temperature shows a temperature of 15 - 30°C unless otherwise specified. The following ¹H-NMR values were measured by a nuclear magnetic resonance apparatus ECP400 (400 MHz) manufactured by JEOL Ltd. As the HPLC apparatus, Shimadzu LC-10ADbp or LC-10A was used. As the GC apparatus, Shimadzu GC-2014 ATF was used.

### Reference Example 1: Synthesis of tafluprost acid

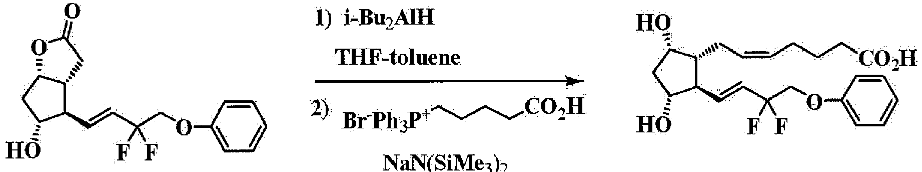

Tetrahydrofuran (1200 g) was added to dissolve (1S,5R,6R,7R)-6-[(1E)-3,3-difluoro-4-phenoxy-1-butenyl]-7-hydroxy-2-oxabicyclo[3.3.0]octan-3-one (280 g) under a nitrogen atmosphere, and diisobutylaluminum hydride (1M toluene solution) (2160 mL) was added dropwise at -70°C. After completion of the dropwise addition, the mixture was stirred for 30 min, 1N hydrochloric acid was added and the mixture was extracted with ethyl acetate. The organic layers were combined, washed with water, and the filtrate was concentrated under reduced pressure to give a reduced form (284 g). To 4-carboxybutyltriphenylphosphonium bromide (1523 g) was added tetrahydrofuran (5030 g) under a nitrogen atmosphere, sodium bis(trimethylsilyl)amide solution (1M tetrahydrofuran solution) (6684 mL) was added dropwise, and the mixture was stirred for 1 hr or longer. The above-mentioned reduced form (286 g) dissolved in tetrahydrofuran (970 g) was added dropwise thereto at 0°C and the mixture was stirred for 3 hr. To the reaction mixture was added water and the mixture was extracted with ethyl acetate. The aqueous layer was acidified, extracted with ethyl acetate, and concentrated under reduced pressure. The insoluble material was filtered off, and the filtrate was purified by silica gel column chromatography (hexane/ethyl acetate=1/1 - 1/3) to give tafluprost acid (222 g). ¹H NMR (CDCl₃) δ 1.60 (m, 1H), 1.67 (m, 2H), 1.84 (m, 1H), 2.02-2.16 (m, 4H), 2.25-2.35 (m, 3H), 2.47 (m, 1H), 4.03 (m, 1H), 4.18 (m, 3H), 5.35-5.42 (m, 2H), 5.80 (m, 1H), 6.10 (m, 1H), 6.91 (m, 2H), 7.00 (m, 1H), 7.30 (m, 2H).

### Reference Example 2: Synthesis of crude product of tafluprost

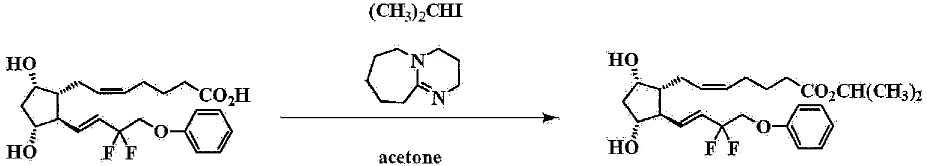

Under a nitrogen atmosphere, tafluprost acid (120 g) obtained in Reference Example 1 was charged in a 5L flask, and dissolved in acetone (600 mL) with stirring. After cooling to 5°C, 1,8-diazabicyclo[5.4.0]undec-7-ene (DBU) (160 mL) was added dropwise while maintaining at 5°C or lower, 2-iodopropane (146 mL) was further added dropwise while maintaining at 5°C or lower, and the mixture was stirred at 30°C until the conversion ratio of the reaction reached 95% or more. To the reaction mixture were added ethyl acetate (1800 mL) and 5% aqueous citric acid solution (900 mL) to allow for partitioning, and the organic layer was washed with 5% aqueous citric acid solution (900 mL, once), 5% aqueous sodium hydrogen carbonate solution (900 mL, twice), and purified water (900 mL, once). Under reduced pressure, the solvent was evaporated at 40°C or less to give a crude product of tafluprost (132 g, yield 100%; HPLC purity: 95.5%, content of α chain trans isomer: 0.73%).

### Example 1

(Step 1) A slurry prepared from silica gel (AGC Si-Tech Co., Ltd., M.S.GEL D50-120A, particle size (d50): 50 µm, spherical, 50 g) and n-hexane/ethanol=96/4 was filled in a column, the crude product of tafluprost (1 g) obtained in Reference Example 2 was dissolved in n-hexane/ethyl acetate=1/1, charged on the column, and eluted with n-hexane/ethanol=96/4. Each collected fraction was analyzed by HPLC, and fractions containing tafluprost were collected. As fractions containing tafluprost, fractions having at least the area percentage of tafluprost of 98% or more (calculated excluding the solvent peak) were collected.

(Step 2) The collected fractions containing tafluprost were concentrated under reduced pressure at 35°C - 40°C.

(Step 3) The residue was dissolved in n-hexane/ethyl acetate=3/2, filtered using a membrane filter (pore size: 0.2 µm), and washed with n-hexane/ethyl acetate=3/2.

(Step 4) The solvent of the filtrate was evaporated at 35°C - 40°C under reduced pressure conditions that make the final ultimate vacuum 1 torr or less for a whole day and night to obtain tafluprost (colorless to pale-yellow viscous oil, yield: 82%, HPLC purity: 99.5%, content of α chain trans isomer: 0.25%).

The residual solvent concentration of the obtained tafluprost was analyzed by GC. As a result, n-hexane was 0 ppm, ethyl acetate was 0 ppm, and ethanol was 0 ppm.

¹H NMR (CDCl₃) δ 1.22 (d, J=6.2 Hz, 3H), 1.22 (d, J=6.2 Hz, 3H), 1.58-1.63 (m, 1H), 1.63-1.69 (m, 2H), 1.84 (d, J=14.7 Hz, 1H), 2.02-2.08 (m, 1H), 2.10-2.16 (m, 3H), 2.25 (t, J=7.3 Hz, 1H), 2.26 (t, J=7.1 Hz, 1H), 2.30-2.35 (m, 1H), 2.46-2.49 (m, 2H), 2.61-2.63 (m, 1H), 4.02-4.03 (m, 1H), 4.18-4.21 (m, 3H), 5.00 (heptet, J=6.2 Hz, 1H), 5.35-5.42 (m, 2H), 5.80 (dt, J=15.8, 11.2 Hz, 1H), 6.10 (dd, J=15.8, 8.8 Hz, 1H), 6.91 (d, J=8.8 Hz, 2H), 7.00 (t, J=7.3 Hz, 1H), 7.30 (dd, J=8.8, 7.3 Hz, 2H); ¹⁹F NMR (CDCl₃) δ -102.8 (dq, ²J_{FF}=255.6 Hz), -103.6 (dq, ²J_{FF}=255.6 Hz).

### <HPLC (reversed phase) analysis conditions>

Column: YMC-Pack ODS-AM (5 µm, 6.0×150 mm)
Temperature: room temperature
Flow rate:1 mL/min
Detection wavelength: 220 nm
Eluent: (SOLUTION A) 1% triethylamine-phosphate buffer (pH 6.3), (SOLUTION B) acetonitrile
Gradient conditions: A/B=50/50 (0 - 45 min), A/B=25/75 (45 - 70 min)

### <GC analysis conditions>

Column: G-column G300 (1.2 mm I.D., 40 m)
Column temperature: 50°C
Detection: hydrogen flame ionization detector
Carrier: helium
Injector temperature: 160°C
Detector temperature: 160°C

### Examples 2 - 6

To investigate the effect of the kind of silica to be used for silica gel column chromatography on the purity and yield of tafluprost, the following experiments were performed in the same manner as in Example 1.

Using a crude product of tafluprost (1 g; HPLC purity: 95.5%) and silica gel (50 g), silica gel column chromatography was performed. The results are shown in the following Table 1. Reversed phase HPLC analysis was performed under the same conditions as the aforementioned conditions.

**[Table 1]**

| Ex. No. | silica gel | shape | particle size (d50) (µm) | eluent (v/v) | HPLC purity (%) | yield (%) |
|---|---|---|---|---|---|---|
| 2 | Merck Silica Gel 60 | crushed | 55-65 | n-hexane/ethyl acetate=2/1 - 3/2 | 98.4 | 38 |
| 3 | Merck Silica Gel 60 | crushed | 55-65 | n-hexane/t-BuOMe=40/60 | 98.5 | 51 |
| 4 | FUJI SILYSIA FL60D | spherical | 60 | n-hexane/ethyl acetate=50/50 | 98.7 | 71 |
| 5 | AGC Si-Tech D50-120A | spherical | 50 | n-hexane/2-propanol=95/5 | 99.3 | 80 |
| 6 | AGC Si-Tech D50-120A | spherical | 50 | n-heptane/ethanol =95/5 | 99.4 | 77 |

### Comparative Example 1

Using a crude product of tafluprost (1 g; HPLC purity: 95.5%), and silica gel (50 g) and eluent used in Example 2, silica gel column chromatography was performed. In the same manner as in Example 1 except that the collected each fraction was analyzed by TLC rather than HPLC, fractions containing tafluprost alone were collected by visual observation. As a result, the HPLC purity of the obtained tafluprost was 97.3%, and did not reach the quality level (limit value: 98%) required for purified pharmaceutical products.

When the particle size (d50) of the silica gel used was 65 µm or less, tafluprost having a purity of more than 98% was obtained in all of Examples 1 - 6 regardless of the difference in shape. Above all, it was found that tafluprost having a particularly high purity can be obtained in a high yield when spherical silica gel is used.

### Example 7 (consideration of scale-up)

### (Step 1)

In the same manner as step 1 in Example 1, a slurry prepared from silica gel (AGC Si-Tech Co., Ltd., M.S.GEL D50-120A, particle size (d50): 50 µm, spherical, 6.0 kg) and n-hexane/ethanol=96/4 was filled in a column, the crude product of tafluprost (120 g) obtained in Reference Example 2 was dissolved in n-hexane/ethyl acetate=1/1, charged on the column, and eluted with n-hexane/ethanol=96/4. Each collected fraction was analyzed by HPLC, and fractions containing tafluprost were collected. As fractions containing tafluprost, fractions having at least the area percentage of tafluprost of 98% or more (calculated excluding the solvent peak) were collected.

### (Steps 2 - 4)

The fractions containing tafluprost, collected in step 1, were concentrated under reduced pressure at 29°C - 35°C (step 2) .

The residue was dissolved in n-hexane/ethyl acetate=3/2, filtered using a membrane filter (pore size: 0.2 µm), and washed with n-hexane/ethyl acetate=3/2 (step 3).

The solvent of the filtrate was evaporated at 32°C - 36°C under reduced pressure conditions that make the final ultimate vacuum 0.30 torr for 26 hr (step 4) to obtain tafluprost (colorless to pale-yellow viscous oil, yield: 86%, HPLC purity: 99.7%, content of α chain trans isomer: 0.24%, microorganism content: not more than 10 cfu/0.1 g).

The residual solvent concentration of the obtained tafluprost was analyzed by GC. As a result, n-hexane was 0 ppm, ethyl acetate was 0 ppm, and ethanol was 0 ppm.

### Example 8

The fractions containing tafluprost, collected in the same manner as step 1 in Example 7, were concentrated under reduced pressure under the conditions similar to those of step 2 in Example 7.

The obtained residue was dissolved in ethyl acetate, filtered using a membrane filter (pore size: 0.2 µm), and washed with ethyl acetate (step 3).

The solvent of the filtrate was evaporated at 23°C - 37°C under reduced pressure conditions that make the final ultimate vacuum 0.26 torr for 27 hr (step 4) to obtain tafluprost (colorless to pale-yellow viscous oil, yield: 85%, HPLC purity: 99.7%, content of α chain trans isomer: 0.27%, microorganism content: not more than 10 cfu/0.1 g).

The residual solvent concentration of the obtained tafluprost was analyzed by GC. As a result, n-hexane was 0 ppm, ethyl acetate was 0 ppm, and ethanol was 0 ppm.

### Example 9

The fractions containing tafluprost, collected in the same manner as step 1 in Example 7, were concentrated under reduced pressure under the conditions similar to those of step 2 in Example 7, and the residue was dissolved in n-hexane/ethyl acetate=3/2, filtered using a membrane filter (pore size: 0.2 µm), and washed with n-hexane/ethyl acetate=3/2 (step 3).

The solvent of the filtrate was evaporated at 20°C - 36°C under reduced pressure conditions that make the final ultimate vacuum 2.6 torr for 3 hr (step 4) to obtain tafluprost (colorless to pale-yellow viscous oil, yield: 75%, HPLC purity: 99.4%, content of α chain trans isomer: 0.30%, microorganism content: not more than 10 cfu/0.1 g).

The residual solvent concentration of the obtained tafluprost was analyzed by GC. As a result, n-hexane was 36 ppm, ethyl acetate was 4803 ppm, and ethanol was 66 ppm.

### Example 10

The fractions containing tafluprost, collected in the same manner as step 1 in Example 7, were concentrated under reduced pressure under the conditions similar to those of step 2 in Example 7, and the residue was dissolved in n-hexane/ethyl acetate=3/2, filtered using a membrane filter (pore size: 0.2 µm), and washed with n-hexane/ethyl acetate=3/2 (step 3).

The solvent of the filtrate was evaporated at 34°C - 37°C under reduced pressure conditions that make the final ultimate vacuum 2.1 torr for 5 hr (step 4) to obtain tafluprost (colorless to pale-yellow viscous oil, yield: 78%, HPLC purity: 99.5%, content of α chain trans isomer: 0.32%, microorganism content: not more than 10 cfu/0.1 g).

The residual solvent concentration of the obtained tafluprost was analyzed by GC. As a result, n-hexane was 2 ppm, ethyl acetate was 785 ppm, and ethanol was 0 ppm.

### Example 11

The fractions containing tafluprost, collected in the same manner as step 1 in Example 7, were concentrated under reduced pressure under the conditions similar to those of step 2 in Example 7, and the residue was dissolved in n-hexane/ethyl acetate=3/2, filtered using a membrane filter (pore size: 0.2 µm), and washed with n-hexane/ethyl acetate=3/2 (step 3).

The solvent of the filtrate was evaporated at 32°C - 36°C under reduced pressure conditions that make the final ultimate vacuum 0.92 torr for 8 hr (step 4) to obtain tafluprost (colorless to pale-yellow viscous oil, yield: 82%, HPLC purity: 99.6%, content of α chain trans isomer: 0.26%, microorganism content: not more than 10 cfu/0.1 g).

The residual solvent concentration of the obtained tafluprost was analyzed by GC. As a result, n-hexane was 0 ppm, ethyl acetate was 86 ppm, and ethanol was 0 ppm.

### Example 12

The fractions containing tafluprost, collected in the same manner as step 1 in Example 7, were concentrated under reduced pressure under the conditions similar to those of step 2 in Example 7, and the residue was dissolved in n-hexane/ethyl acetate=3/2, filtered using a membrane filter (pore size: 0.2 µm), and washed with n-hexane/ethyl acetate=3/2 (step 3).

The solvent of the filtrate was evaporated at 35°C - 39°C under reduced pressure conditions that make the final ultimate vacuum 0.09 torr for 50 hr (step 4) to obtain tafluprost (colorless to pale-yellow viscous oil, yield: 80%, HPLC purity: 99.5%, content of α chain trans isomer: 0.26%, microorganism content: not more than 10 cfu/0.1 g).

The residual solvent concentration of the obtained tafluprost was analyzed by GC. As a result, n-hexane was 0 ppm, ethyl acetate was 0 ppm, and ethanol was 0 ppm.

### Example 13

The fractions containing tafluprost, collected in the same manner as step 1 in Example 7, were concentrated under reduced pressure under the conditions similar to those of step 2 in Example 7, and the residue was dissolved in n-hexane/ethyl acetate=3/2, filtered using a membrane filter (pore size: 0.2 µm), and washed with n-hexane/ethyl acetate=3/2 (step 3).

The solvent of the filtrate was evaporated at 36°C - 45°C under reduced pressure conditions that make the final ultimate vacuum 0.24 torr for 60 hr (step 4) to obtain tafluprost (colorless to pale-yellow viscous oil, yield: 79%, HPLC purity: 99.5%, content of α chain trans isomer: 0.26%, microorganism content: not more than 10 cfu/0.1 g).

The residual solvent concentration of the obtained tafluprost was analyzed by GC. As a result, n-hexane was 0 ppm, ethyl acetate was 0 ppm, and ethanol was 0 ppm.

The solvent evaporation conditions, tafluprost yield, purity and content of α chain trans isomer, and the results of residual solvent concentration in the above-mentioned Examples 7 - 13 are shown in the following Table 2.

**[Table 2]**

| | Ex. 7 | Ex. 8 | Ex. 9 | Ex. 10 | Ex. 11 | Ex. 12 | Ex. 13 |
|---|---|---|---|---|---|---|---|
| temperature (°C) during concentration under reduced pressure | 32-36 | 23-37 | 20-36 | 34-37 | 32-36 | 35-39 | 36-45 |
| final ultimate vacuum (torr) | 0.3 | 0.26 | 2.6 | 2.1 | 0.92 | 0.09 | 0.24 |
| reduced pressure time (hr) | 26 | 27 | 3 | 5 | 8 | 50 | 60 |
| | | | | | | | |
| yield (%) | 86 | 85 | 75 | 78 | 82 | 80 | 79 |
| HPLC purity (%) | 99.7 | 99.7 | 99.4 | 99.5 | 99.6 | 99.5 | 99.5 |
| isomer content (%) | 0.24 | 0.27 | 0.3 | 0.32 | 0.26 | 0.26 | 0.26 |
| | | | | | | | |
| n-hexane (ppm) | 0 | 0 | 36 | 2 | 0 | 0 | 0 |
| ethyl acetate (ppm) | 0 | 0 | 4803 | 785 | 86 | 0 | 0 |
| ethanol (ppm) | 0 | 0 | 66 | 0 | 0 | 0 | 0 |

According to Table 2, tafluprost was obtained with good purity and high yield in all of Examples 7 - 13, the residual organic solvent concentration can be suppressed below the concentration limit value indicated in the Residual Solvent Guideline for Pharmaceutical Products, and it was found that the purification method of the present invention is a purification method that can withstand scale-up and has broad utility. On the other hand, in Comparative Example in which concentration under reduced pressure was performed when the final ultimate vacuum was 8 torr, it was confirmed that the residual organic solvent concentration exceeds the concentration limit value indicated in the Residual Solvent Guideline for Pharmaceutical Products.

### Experimental Example

### Consideration of heat stability of tafluprost

About 120 mg of tafluprost obtained in Example 1 was weighed in a glass container and stored in a thermostatic tank at 40°C, quantified by reversed phase HPLC analysis, and changes in the tafluprost content over time were examined. Similarly, about 20 mg of tafluprost was weighed in a glass container and stored in a thermostatic tank at 60°C or 80°C, and changes in the tafluprost content over time were examined.

### <HPLC (reversed phase) analysis conditions>

Column: YMC-Pack ProC18 AS-303 (5 pm, 4.6x250 mm)
Temperature: 50°C
Flow rate: 1 mL/min
Detection wavelength: 220 nm
Eluent: (SOLUTION A) 10 mmol/L phosphoric acid (sodium) buffer (pH 6.9), (SOLUTION B) acetonitrile
Gradient conditions: A/B=50/50 (0 - 45 min), A/B=25/75 (45 - 70 min)

The examination results of the changes in the tafluprost content over time under each temperature are shown in the following Tables 3 - 5.

**[Table 3]**

| Stability of tafluprost at 40°C | | | |
|---|---|---|---|
| time (month) | 0 | 3 | 6 |
| tafluprost content (%) | 101.3 | 99.8 | 99.4 |

**[Table 4]**

| Stability of tafluprost at 60°C | | | | |
|---|---|---|---|---|
| time (day) | 0 | 3 | 7 | 14 |
| tafluprost content (%) | 99.0 | 98.9 | 98.5 | 95.4 |

**[Table 5]**

| Stability of tafluprost at 80°C | | | | |
|---|---|---|---|---|
| time (day) | 0 | 1 | 3 | 7 |
| tafluprost content (%) | 99.5 | 99.1 | 94.4 | 86.7 |

According to the results in Tables 3 - 5, it was confirmed that tafluprost gradually decomposes with the passage of time at a temperature of 60°C or more, and particularly remarkably decomposes at 80°C, even during a storage period of several days to 2 weeks. However, it was found that tafluprost stably exists at 40°C even after 6 months.

From the above results, in the purification method of the present invention, contamination by impurities (analogues) derived from the decomposition of tafluprost can be suppressed by performing concentration under reduced pressure or evaporation of solvent at a temperature of 55°C or less (particularly preferably 45°C or less).

### [Industrial Applicability]

According to the purification method of the present invention, the contamination by impurities can be minimized by collecting fractions containing tafluprost by HPLC analysis during separation and purification of a crude product of tafluprost by silica gel column chromatography in the final step of tafluprost production. In addition, the concentration of the residual organic solvent can be suppressed below the concentration limit value indicated in the Residual Solvent Guideline for Pharmaceutical Products by evaporating the solvent over time under reduced pressure conditions of low temperature and high vacuum level, and the decomposition of tafluprost which is unstable under high temperature can also be suppressed. Furthermore, fine powder of silica gel, airborne particles in the air, and bacteria can be removed by incorporating a filter filtration step thereinto. Thus, the method has the advantage that, after evaporation of the solvent, it is possible to easily and efficiently provide highly pure tafluprost that can be used as it is as a drug substance of pharmaceutical products. In addition, the purification method of the present invention can be widely applied to the crude products of tafluprost produced by known methods, can withstand scale-up, and has broad utility.

This application is based on a patent application No. 202110111991.0 filed in People's Republic of China (filing date: January 27, 2021), the contents of which are incorporated in full herein.

## Claims

1. A method for purifying tafluprost, comprising a step of purifying a crude product of tafluprost by silica gel column chromatography, and collecting a fraction containing tafluprost by HPLC analysis.

2. The purification method according to claim 1, further comprising a step of concentrating the tafluprost-containing fraction collected by the HPLC analysis under reduced pressure at 10 - 55°C, a step of dissolving the residue in a solvent and filtering the solution, and a step of evaporating the solvent from the filtrate at 10 - 55°C under reduced pressure that affords a final ultimate vacuum of 5 torr or less.

3. The purification method according to claim 1 or 2, wherein a particle size (d50) of silica gel used for the silica gel column chromatography is 20 - 70 µm.

4. The purification method according to any one of claims 1 to 3, wherein the silica gel used for the silica gel column chromatography is spherical.

5. The purification method according to any one of claims 1 to 4, wherein an eluent of the silica gel column chromatography is a mixed solvent of n-hexane and a polar solvent, or a mixed solvent of n-heptane and a polar solvent.

6. The purification method according to claim 5, wherein the eluent is a mixed solvent of n-hexane and a polar solvent.

7. The purification method according to claim 5 or 6, wherein the polar solvent is ethyl acetate, t-butyl methyl ether, 2-propanol, or ethanol.

8. The purification method according to any one of claims 1 to 7, wherein the HPLC analysis is reversed phase HPLC analysis.

9. The purification method according to any one of claims 1 to 8, wherein the fraction comprises tafluprost in a proportion of 98% or more.

10. The purification method according to any one of claims 2 to 9, wherein the filtration is performed using a filter having a pore size of 0.5 µm or less.

11. The purification method according to any one of claims 2 to 10, wherein the solvent used for dissolving the residue is ethyl acetate, t-butyl methyl ether, 2-propanol or ethanol, or a mixed solvent of a nonpolar solvent and ethyl acetate, t-butyl methyl ether, 2-propanol or ethanol.

12. The purification method according to claim 11, wherein the solvent used for dissolving the residue is ethyl acetate or a mixed solvent of ethyl acetate and a nonpolar solvent.

13. The purification method according to claim 11 or 12, wherein the nonpolar solvent is n-hexane or n-heptane.

14. The purification method according to any one of claims 2 to 13, wherein the final ultimate vacuum is 1 torr or less.

15. The purification method according to any one of claims 2 to 14, wherein, after the step of evaporating the solvent from the filtrate, the residual solvent concentration of n-hexane is 290 ppm or less, and the residual solvent concentration of each of n-heptane, ethyl acetate, t-butyl methyl ether, 2-propanol and ethanol is 5000 ppm or less.

16. A method for producing tafluprost, comprising a step of subjecting a crude product of tafluprost to the purification method according to any one of claims 1 to 15.

17. A tafluprost obtained by the production method according to claim 16.

18. A medicament comprising the tafluprost according to claim 17 as an active ingredient.

19. A medicament for the prophylaxis or treatment of an ophthalmic disease, comprising the tafluprost according to claim 17 as an active ingredient.

20. The medicament according to claim 19, wherein the ophthalmic disease is glaucoma or an ocular hypertension.
